# EUROPEAN PATENT SPECIFICATION

(11) **EP 4 215 141 B1**
(45) Date of publication and mention of the grant of the patent: **29.04.2026**
(21) Application number: 23152472.9
(22) Date of filing: 19.01.2023
(51) Int. Cl.: A61B 18/14

(54) **SYSTEMS AND METHODS FOR LINEAR SPINES FORMING A SPHERICAL BASKET FOR IMPROVED TISSUE CONTACT AND CURRENT DELIVERY**
SYSTEME UND VERFAHREN FÜR LINEARE DORNE ZUR FORMUNG EINES KUGELFÖRMIGEN KORBS FÜR VERBESSERTEN GEWEBEKONTAKT UND VERBESSERTE STROMABGABE
SYSTÈMES ET PROCÉDÉS POUR DES COLONNES LINÉAIRES FORMANT UN PANIER SPHÉRIQUE POUR UN CONTACT TISSULAIRE AMÉLIORÉ ET UNE DISTRIBUTION DE COURANT AMÉLIORÉE

(30) Priority: 20.01.2022 US 202263301102 P; 14.12.2022 US 202218065748
(43) Date of publication of application: 26.07.2023
(73) Proprietor: Biosense Webster (Israel) Ltd, 2066717 Yokneam (IL)
(72) Inventor: OKARSKI, Kevin Mark, Irvine 92618 (US); DATTA, Keshava, Irvine 92618 (US); BAH, Abubakarr, Irvine 92618 (US); NGUYEN, Thanh, Irvine 92618 (US)
(74) Representative: Carpmaels & Ransford LLP

(56) References cited:
- US-A1- 2002 198 522
- US-A1- 2013 090 651
- US-A1- 2015 011 991
- US-A1- 2015 351 652
- US-A1- 2017 035 496
- US-A1- 2017 319 140

## Description

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation but may present tissue damage due to the very low temperature nature of such devices. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly and alternative catheter geometries in general.

US 2002/0198522 A1 discloses an electrode support structure that has a slotted hub and an integral body with a mid-section and opposed pair of spline elements that extend from the mid-section. The mid-section is captured within the slot, securing the integral body to the hub with the opposed spline elements radiating free of the slot for carrying one or more electrodes.

US2013/0090651 A1 discloses systems for nerve modulation through the wall of a blood vessel. An example system for nerve modulation may include an elongate member extending along a central elongate axis and having a proximal end and a distal end. The elongate member may have a radially expandable member disposed proximate the distal end. A tubular sheath may be cooperatively engaged with the expandable member such that the expandable member is collapsed when in the sheath and can expand when moved distally relative to and past a distal end of the sheath. The expandable member may include a plurality of electrodes and a plurality of spacer struts. Each spacer strut may be configured such that when the self-expanding member is in an expanded state the spacer strut extends out radially further than the electrodes from the central elongate axis.

### SUMMARY

A medical probe and a method of construction of the same are provided in accordance with claims 1 and 7 hereinafter. Further embodiments are provided in the dependent claims.

### BRIEF DESCRIPTION OF THE DRAWINGS

FIG. 1 is a schematic pictorial illustration of a medical system including a medical probe whose distal end includes a basket assembly with electrodes, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, , in accordance with embodiments of the present invention;
FIG. 2C is a schematic pictorial illustration showing an exploded side view of a medical probe, in accordance with an embodiment of the present invention;
FIGs. 3A and 3B are schematic pictorial illustrations showing a profile outline of a basket assembly of a given medical device, in accordance with embodiments of the present invention;
FIG. 4 is a schematic pictorial illustration showing a side view of a plurality of linear spines forming a basket assembly, in accordance with an embodiment of the present invention;
FIGs. 5A and 5B are schematic pictorial illustrations of a method of forming a basket assembly, in accordance with an embodiment of the present invention;
FIG. 5C illustrates an embodiment where the proximal end of each spine is provided with a hole and reference notches to ensure correct alignment and retention of the spine to the irrigation tube, in accordance with an embodiment of the present invention;
FIG. 5D illustrates an embodiment that relies on a balloon to expand the spine assembly, in accordance with an embodiment of the present invention;
FIG. 5E illustrates a spine assembly formed by cutting a cylindrical tube stock with a laser, in accordance with an embodiment of the present invention;
FIG. 5F illustrates a spine assembly after shape setting of the spines in Fig. 5E into a spheroidal basket like shape, in accordance with an embodiment of the present invention;
FIGs. 6A through 6E are schematic pictorial illustrations of central spine intersections;
FIGs. 7A through 7J are schematic pictorial illustrations showing a perspective view of various example electrodes, in accordance with embodiments of the present invention;
FIGs. 8A and 8B are schematic pictorial illustrations showing various insulative jackets of a given medical device, in accordance with embodiments of the present invention;
FIGs. 9A and 9B are schematic pictorial illustrations showing cross-sectional views of a given wire of a medical probe, in accordance with an embodiment of the present invention;
FIG. 10 is a schematic pictorial illustration of a method of cutting a plurality of linear spines from a planar sheet of material, in accordance with an embodiment of the present invention;
FIGs. 11A through 11D are schematic pictorial illustrations of a method of cutting a plurality of linear spines from a planar sheet of material, in accordance with an embodiment of the present invention;
FIGs. 12A and 12B are schematic pictorial illustrations of a method of cutting a plurality of linear spines including one or more cutouts at a central spine intersection from a planar sheet of material, in accordance with an embodiment of the present invention; and
FIG. 13 is a flowchart illustrating another method of assembling a basket assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors including electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation are disclosed in U.S. Patent Publication 2021/0162210.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1.

To deliver pulsed field ablation (PFA) in an IRE (irreversible electroporation) procedure, electrodes should contact the tissue being ablated with a sufficiently large surface area. As described hereinbelow, the medical probe includes a tubular shaft including proximal and distal ends, and a basket assembly at the distal end of the tubular shaft. The basket assembly includes a single unitary structure. The unitary structure can include a plurality of linear spines formed from a planar sheet of material and one or more electrodes coupled to each of the spines. The plurality of linear spines can converge at a central spine intersection including one or more cutouts. The cutouts can allow for bending of each spine such that the spines form an approximately spherical or oblate-spheroid basket assembly. It is noted that the cutouts (in various configurations described and illustrated in the specification) allows the basket to be compressed into a much smaller form factor when undeployed (or undergoing a retraction into a delivery sheath) without buckling or plastic deformation.

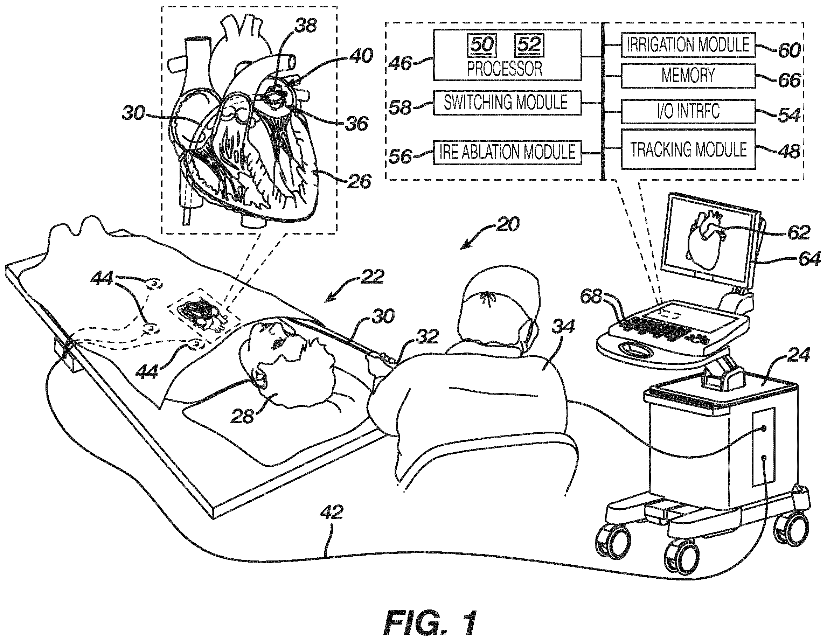

FIG. 1 is a schematic, pictorial illustration of a medical system 20 including a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

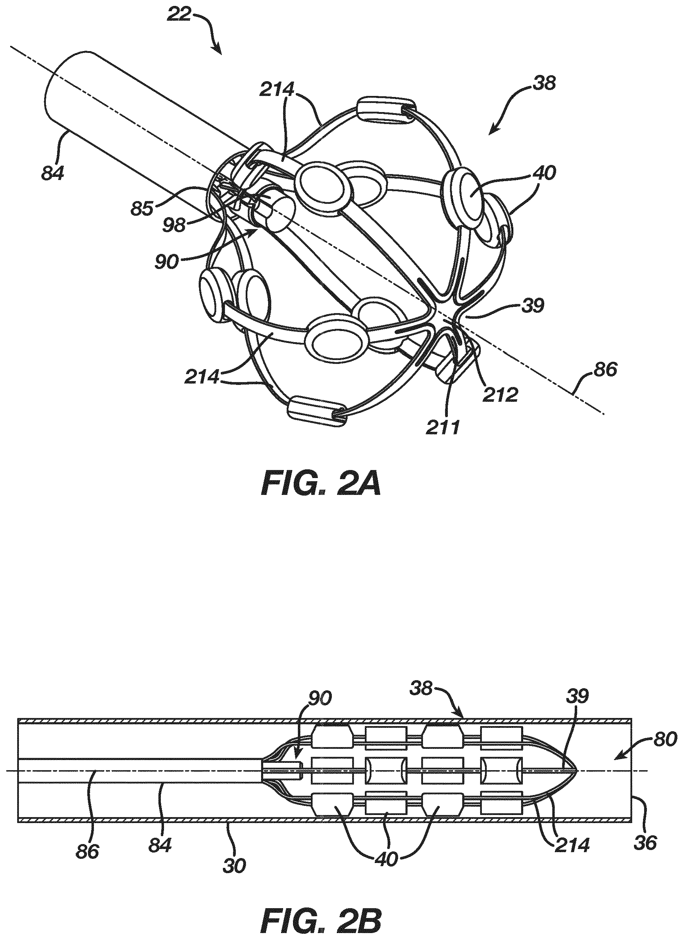

Medical probe 22 includes a flexible insertion tube 30 and a handle 32 coupled to a proximal end of the tubular shaft. During a medical procedure, a medical professional 34 can insert probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 approximate a distal end 36 of the medical probe 22. Basket assembly 38 can include a plurality of electrodes 40 affixed to a plurality of spines 214, as described in the description referencing FIGs. 2A and 2B hereinbelow. To start performing a medical procedure such as irreversible electroporation (IRE) ablation, medical professional 34 can manipulate handle 32 to position distal end 36 so that electrodes 40 engage cardiac tissue at a desired location or locations. Upon positioning the distal end 36 so that electrodes 40 engages cardiac tissue, the medical professional 34 can activate the medical probe 22 such that electrical pulses are delivered by the electrodes 40 to perform the IRE ablation.

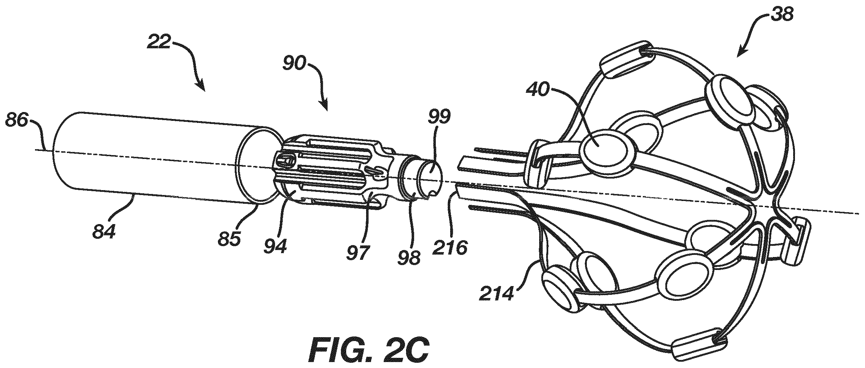

The medical probe 22 can include a guide sheath and a therapeutic catheter, wherein the guide sheath includes the flexible insertion tube 30 and the handle 32 and the therapeutic catheter includes the basket assembly 38, electrodes 40, and a tubular shaft 84 (see FIGs. 2 through 4). The therapeutic catheter is translated through the guide sheath so that the basket assembly 38 is positioned in the heart 26. The distal end 36 of the medical probe 22 corresponds to a distal end of the guide sheath when the basket assembly 38 is contained within the flexible insertion tube 30, and the distal end 36 of the medical probe 22 corresponds to a distal end of the basket assembly 38 when the basket assembly 38 is extended from the distal end of the guide sheath. The medical probe 22 can be alternatively configured to include a second handle on the therapeutic catheter and other features as understood by a person skilled in the pertinent art.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are preferably configured to deliver IRE ablation energy to tissue in heart 26, configuring electrodes 40 to deliver any other type of ablation energy to tissue in any body cavity is possible. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses including peak power in the range of tens of kilowatts. In some examples, the electrodes 40 are configured to deliver electrical pulses including a peak voltage of at least 900 volts (V). The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

In order to dissipate the heat and to improve the efficiency of the ablation process, system 20 supplies irrigation fluid (e.g., a saline solution) to distal end 36 and to the electrodes 40 via a channel (not shown) in tubular shaft 84 (see FIGs. 2A through 2C). Additionally, or alternatively, irrigation fluid can be supplied through the flexible insertion tube 30. Control console 24 includes an irrigation module 60 to monitor and control irrigation parameters, such as the pressure and the temperature of the irrigation fluid. It is noted that while the preference for the exemplary embodiments of the medical probe is for IRE or PFA, it is possible to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some embodiments, medical professional 34 can manipulate map 62 using one or more input devices 68. In alternative embodiments, display 64 may include a touchscreen that can be configured to accept inputs from medical professional 34, in addition to presenting map 62.

FIG. 2A is a schematic pictorial illustration showing a perspective view of a medical probe 22 including a basket assembly 38 in an expanded form when unconstrained, such as by being advanced out of an insertion tube lumen 80 at a distal end 36 of an insertion tube 30. The medical probe 22 illustrated in FIG. 2A lacks the guide sheath illustrated in FIG. 1. FIG. 2B shows the basket assembly in a collapsed form within insertion tube 30 of the guide sheath. In the expanded form (FIG. 2A), spines 214 bow radially outwardly and in the collapsed form (FIG. 2B) the spines are arranged generally along a longitudinal axis 86 of insertion tube 30.

As shown in FIG. 2A, basket assembly 38 includes a plurality of flexible spines 214 that are formed at the end of a tubular shaft 84 and are connected at both ends. During a medical procedure, medical professional 34 can deploy basket assembly 38 by extending tubular shaft 84 from insertion tube 30 causing basket assembly 38 to exit insertion tube 30 and transition to the expanded form. Spines 214 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

As shown in FIG. 2A, the plurality of flexible linear spines 214 converge at a central spine intersection 211. In some examples central spine intersection 211 can include one or more cutouts 212 that allow for bending of the spines 214 when each spine respective attachment end 216 is connected to the spine retention hub 90, described in more detail below.

In embodiments described herein, one or more electrodes 40 positioned on spines 114 of basket assembly 38 can be configured to deliver ablation energy (RF and/or IRE) to tissue in heart 26. Additionally, or alternatively, the electrodes can also be used to determine the location of basket assembly 38 and/or to measure a physiological property such as local surface electrical potentials at respective locations on tissue in heart 26. The electrodes 40 can be biased such that a greater portion of the one or more electrodes 40 face outwardly from basket assembly 38 such that the one or more electrodes 40 deliver a greater amount of electrical energy outwardly away from the basket assembly 38 (i.e., toward the heart 26 tissue) than inwardly.

Examples of materials ideally suited for forming electrodes 40 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 26.

Basket assembly 38 has a distal end 39. The medical probe 22 can include a spine retention hub 90 that extends longitudinally from a distal end of tubular shaft 84 towards distal end 39 of basket assembly 38. As described supra, control console 24 includes irrigation module 60 that delivers irrigation fluid to basket assembly 38 through tubular shaft 84.

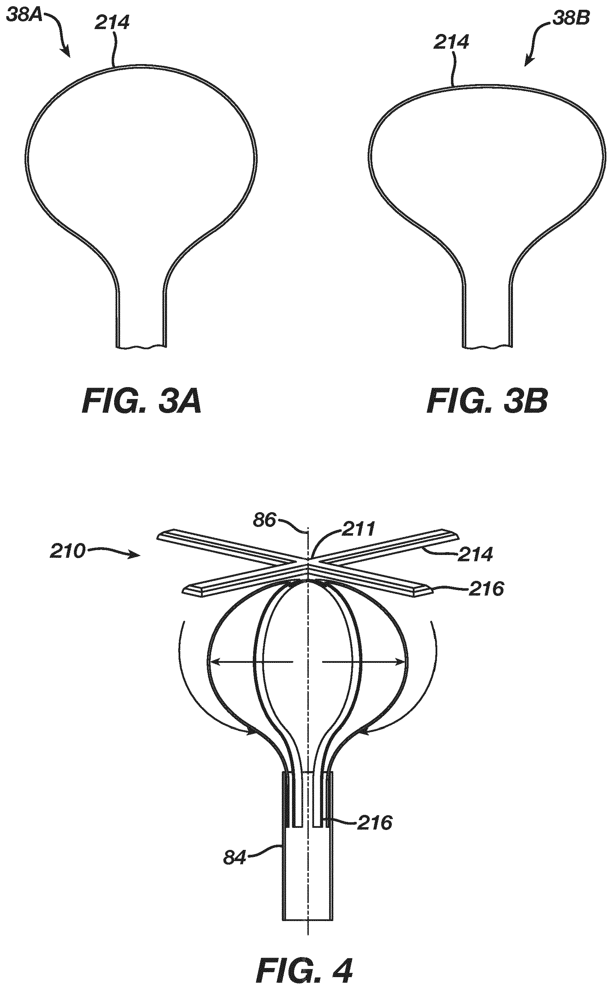

Turning to FIG. 2C, basket assembly 38 includes a single unitary structure that includes a plurality of linear spines 214 formed from a planar sheet of material 210 (shown more clearly in FIGs. 3 and 4A). The spine retention hub 90 can be inserted into the tubular shaft 84 and attached to the tubular shaft 84. Spine retention hub 90 can include a cylindrical member 94 including a plurality of relief lands 96, multiple irrigation openings 98, and at least one spine retention hub electrode 99, or some combination thereof. Relief lands 96 can be disposed on the outer surface of cylindrical member 94 and configured to allow a portion of each spine 214, such as each spine attachment end 216, to be fitted into a respective relief land 96. The attachment end 216 can be a generally linear end of the spine 214. The attachment end 216 can be configured to extend outwardly from the spine retention hub 90 such that the basket assembly 38 is positioned outwardly from the spine retention hub 90 and, consequently, outwardly from the tubular shaft 84. In this way, the spine 214 can be configured to position the basket assembly 38 distally from the distal end of the tubular shaft 84 and distal from the distal end of the insertion tube 30 when the basket assembly is deployed.

As described supra, control console 24 includes irrigation module 60 that delivers irrigation fluid to distal end 36. The multiple irrigation openings 98 can be angled to spray or otherwise disperse of the irrigation fluid to either a given electrode 40 or to tissue in heart 26. Since electrodes 40 do not include irrigation openings that deliver irrigation fluid, the configuration described hereinabove enables heat to be transferred from the tissue (i.e., during an ablation procedure) to the portion of the electrodes on the inner side of the spines 214, and the electrodes 40 can be cooled by aiming the irrigation fluid, via irrigation openings 98, at the portion of the electrodes 40 on the inner side of the spines 214. Spine retention hub electrode 99 disposed at a distal end of retention hub 90 can be used in combination with electrodes 40 on the spines 214, or alternatively, can be used independently from electrodes 40 for reference mapping or ablation.

FIGs. 3A and 3B are schematic pictorial illustrations showing a profile outline of a basket assembly 38A, 38B such that when the basket assembly is deployed the spines define a three-dimensional shape including the profile. The basket assembly can be approximately spheroid including an approximately circular profile as shown in FIG. 3A. The basket assembly can have an approximately oblate-spheroid shape including an approximately elliptical profile as shown in FIG. 3B. Although not every variation of shape is shown or described herein, one skilled in the art will appreciate that spines 214 can be further configured to form other various shapes as would be suitable for the particular application.

By including spines 214 configured to form various shapes when in the expanded form, basket assembly 38 can be configured to position the various electrodes 40 attached to spines 214 at various locations, with each location being nearer or farther from the distal end of tubular shaft 84. For example, electrode 40 attached to spine 214 illustrated in FIG. 3A near the middle of spine 214 would be farther from the distal end of tubular shaft 84 than spine 214 illustrated in FIG. 3B when basket assembly 38 is in the expanded form. In addition, each spine 214 may have an elliptical (e.g., circular) or rectangular (that may appear to be flat) cross-section, and include a flexible, resilient material (e.g., a shape-memory alloy such as nickel-titanium (also known as Nitinol), cobalt chromium, or any other suitable material).

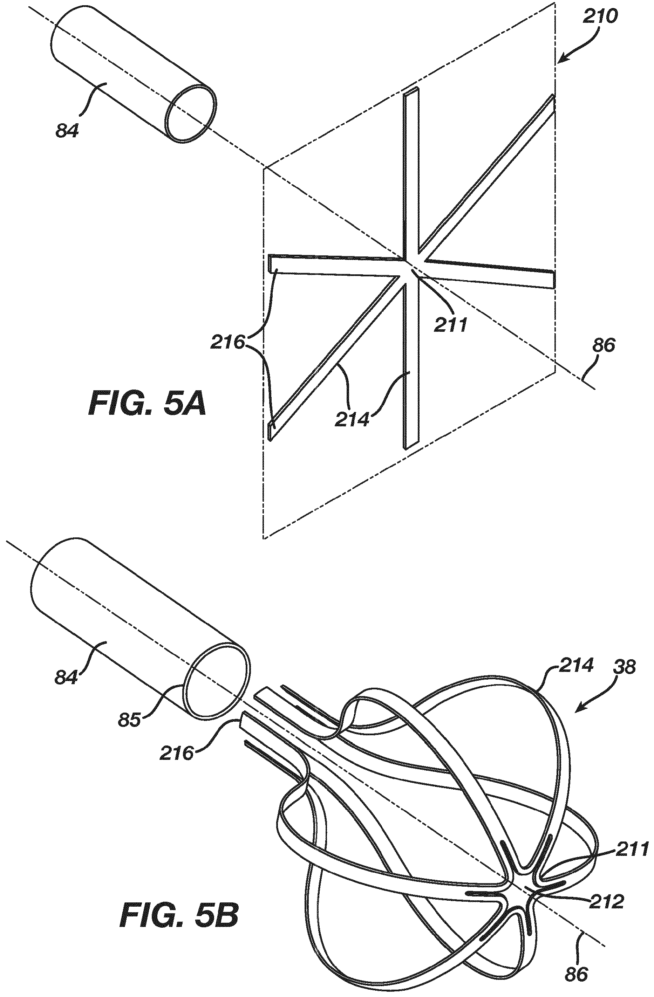

FIGs. 4, 5A and 5B are schematic pictorial illustrations showing views of spines 214 forming basket assembly 38. FIG. 4 provides one example of how planar sheet of material 210 may be assembled together with tubular shaft 84 whereby each spine 214 bends or curves when respective attachment ends 216 are connect to spine retention hub 90. As shown in FIG. 5A, the spines 214 can be formed from a single sheet of planar material 210 to form a generally star shape. In other words, spines 214 can be formed from the single sheet of planar material such that the spines 214 converge toward a central intersection 211. The intersection 211 can be a solid piece of material (as shown in FIG. 5A) or include one or more cutouts 212 (as shown in FIG. 5B). Basket assembly 38 can include a number of spines 214 ranging from about four to about ten spines from a single sheet of planar material 210.

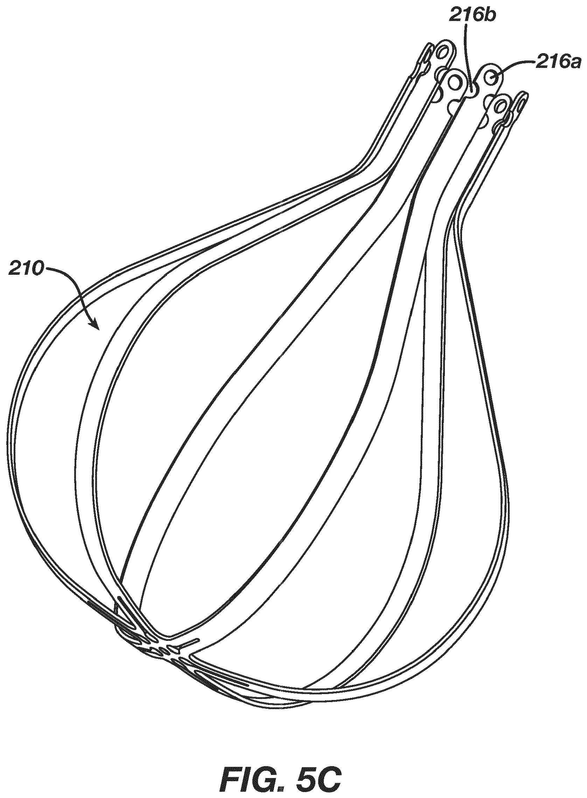

The spine assembly 210 can be physically connected to the tubular member 84 via a suitable technique such as adhesive or molding. In one embodiment shown here in Fig. 5C, eyelet 216a as well as locators 216b can be provided to aid in assembly as well as physical retention of the spines to the tubular member 84.

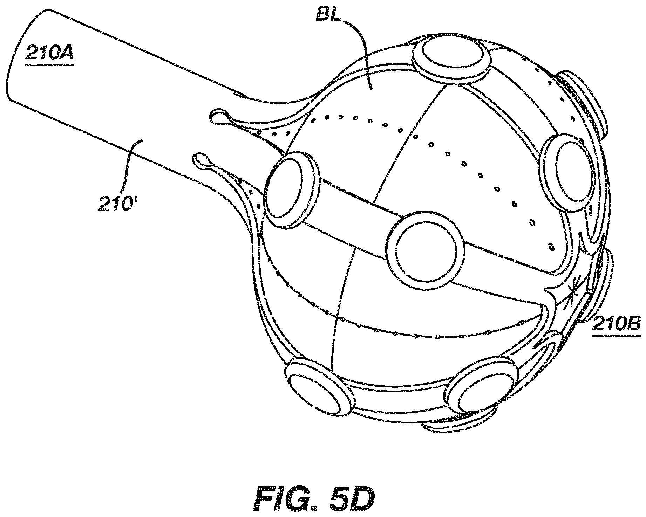

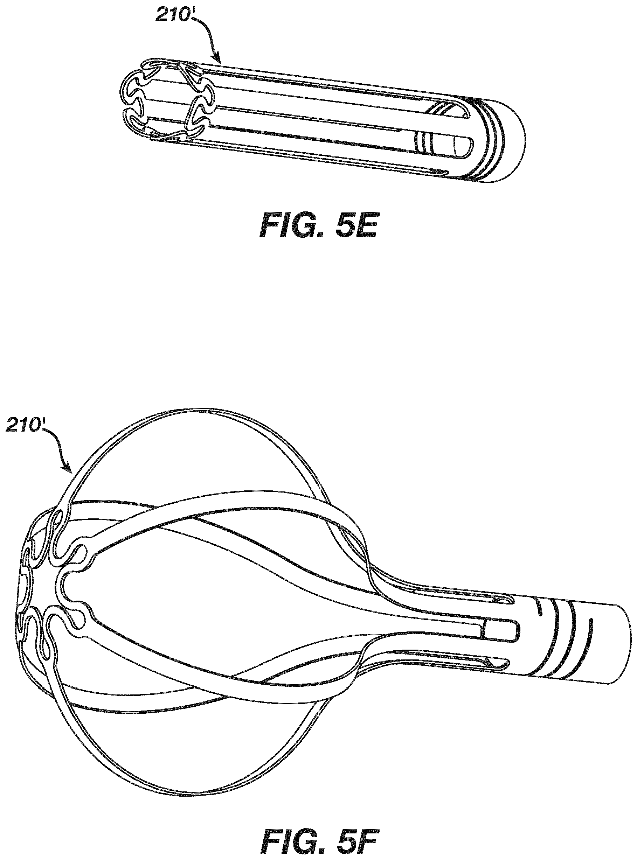

Where it is desired, a balloon BL can be provided as shown in Fig. 5D inside the spine assembly 210' to ensure full expansion of the spine assembly 210' from a cylindrical form factor into a spheroidal form as shown in Fig. 5C. In the embodiment of Fig. 5C, the spine assembly can be made from a tubular cylindrical stock material so that the proximal portion 210A and distal portion 210B are of one-piece material. The tubular stock is cut into a desired shape for the spine assembly 210' as shown in Fig. 5E. Thereafter, the cut tube can be shape set (or heat set) as is known by those skilled in the art to provide for the spheroidal spine configuration shown in Fig. 5F.

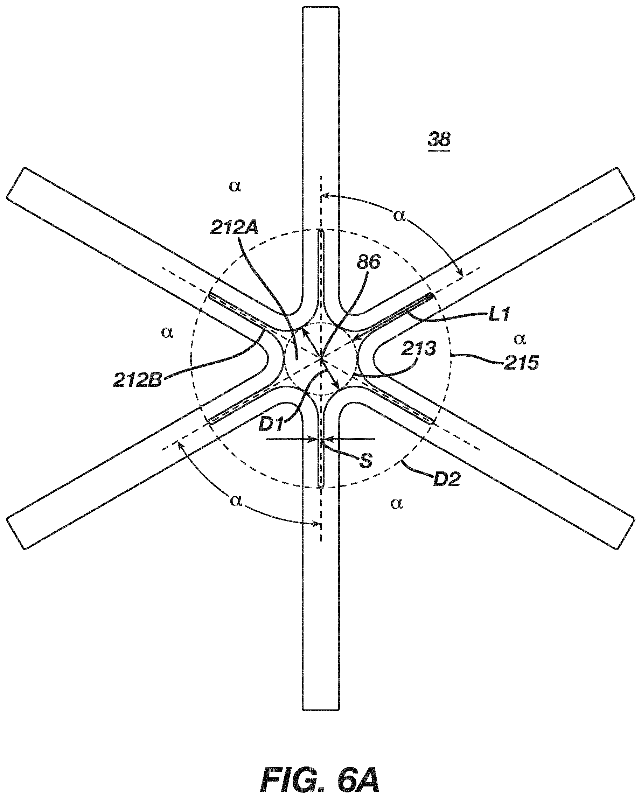

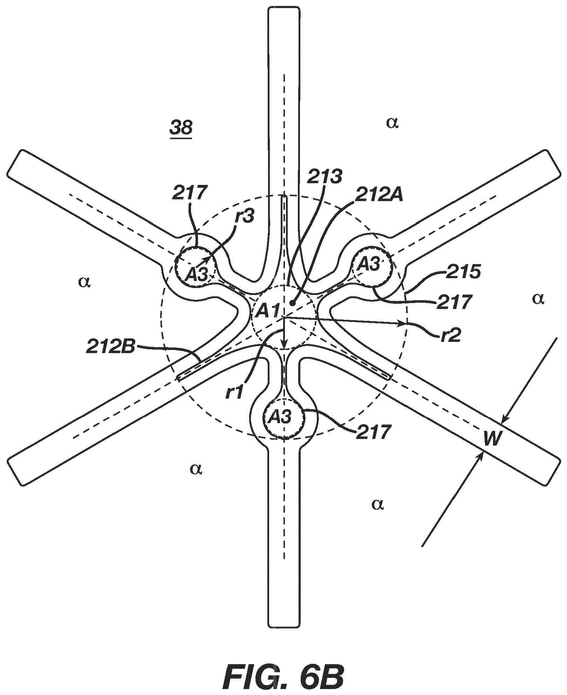

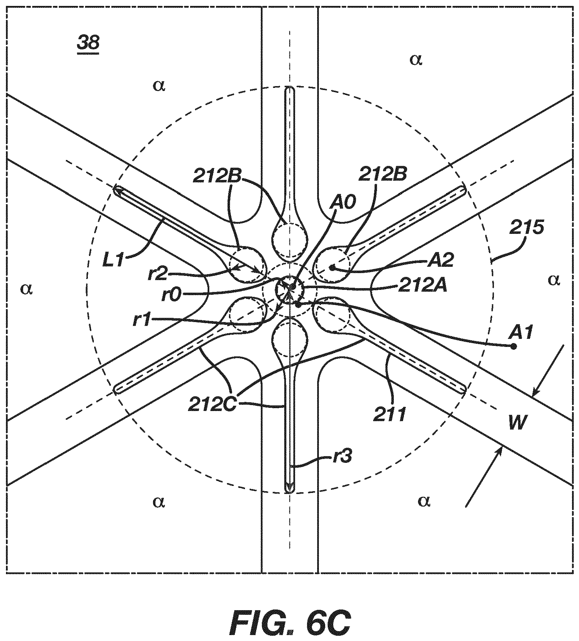

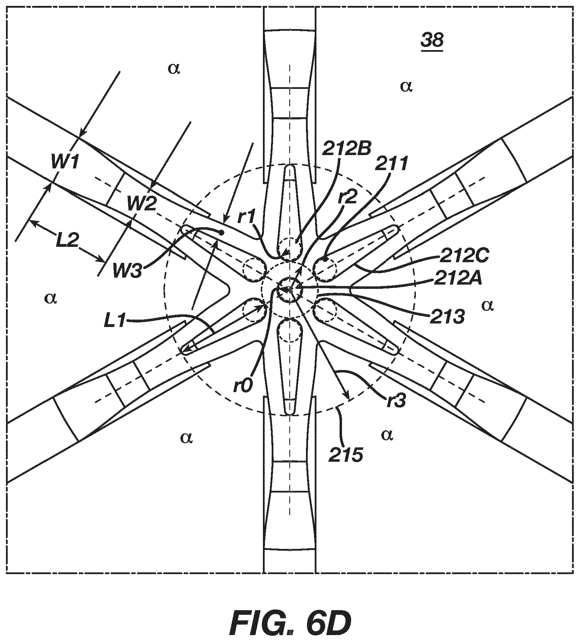

FIGs. 6A through 6E are schematic pictorial illustrations of top-down views of basket assembly 38, showing various examples of one or more cutouts 212 on central spine intersection 211. As shown, intersection 211 can include a single discrete cutout 212A, as shown in FIGs. 6A and 6B. Alternatively, intersection 211 can include two or more cutouts 212B, as provided as an example in FIGs. 6C and 6D. The one or more cutouts 212A, 212B can include a variety of patterns, such as centrosymmetric (i.e., symmetric with respect to a central point), and equiangular (i.e., including equal angles) to allow for equal bending among the spines 214 as well as disproportional and asymmetric to allow for unequal bending of spines 214 to alter structural stability. In certain instances, when basket assembly 38 includes an even number of spines 214, the pattern of the one or more cutouts 212 can alter between every other spine, as illustrated in FIG. 6B. In some examples, one or more cutouts 212 can extend along a portion of each spine 214. Each of the designs illustrated in FIGS. 6A-6E will be discussed separately.

In FIG. 6A, the distal end of the basket assembly 38 has an open cutout 212 which is a combination of a central opening 212A (substantially approximated by a virtual circle 213 with diameter D1) and the groove 212B for each spine (giving a total of six grooves 212B). Each spine is disposed generally equiangularly about the longitudinal axis of a predetermined angle α between any two spines. Each groove 212B has groove width S that extend approximately a length L1 from the circumference of virtual circle D1 so that a virtual circle 215 with diameter D2 is contiguous to the grooves 212B. The second virtual circle 215 has a diameter D2 approximately 3.6 times that of the diameter D1 of the first virtual circle 213. In one embodiment, the cut-out 212 (represented by center cut-out 212A and open grooves 212B) has a negative area of about 1.9 mm-squared with the diameter of the virtual circle 213 of about 1.1 mm and the virtual circle 215 of about 4mm such that each open groove 212B has a width S of about 0.08mm extending for about 1.5mm from the virtual circle 213 so that the negative area defined by all the cut-outs in this design includes approximately 1.9mm-squared.

In FIG. 6B, the basket 38 has its distal portion configured to have an open center 212A that radiates into each of the six spines 214. The open center 212A has a first area A1 that can be approximated by a virtual circle with radius r1. Three spines approximately 120 degrees apart have tapering grooves 212B extending back toward the proximal portion of basket 38. Three other spines approximately 120 degrees apart have large apertures 217 with area A3 disposed towards the proximal portion of the basket 38. The cut-out area A3 can be approximated by a virtual circle with radius r3 and disposed on the spines 214 such that the apertures 217 are contiguous to the inside circumference of virtual circle 215 with radius r2. In this configuration, each third area A3 is about 1/4 of the open first area A1 while the total negative surface area of the entire cut-out includes approximately 1.6 times the first open area of empty space A1 and the second area A2 (calculated with radius r2) includes approximately 7 times the first area A1. Additionally, the second area A2 includes approximately 36 times third area A3. The radius r3 includes approximately 0.4 times that of radius r1 while radius r2 includes approximately 2.8 times that of radius r1. In one exemplary embodiment, first open area of empty space A1 includes approximately 2 mm-squared; second area A2 (as defined by radius r2) being approximately 15mm-squared; third area A3 includes approximately 0.4 mm-squared; total area of all cut-outs includes approximately 3.5 mm-squared; radius r1 ~ 0.8mm; r2 ~2.2mm; and r3 ~0.4mm.

In FIG. 6C, this design has a small aperture 212A disposed at the center (coincident with longitudinal axis 86) of the basket 38 with a tadpole shaped cut-out 211 disposed on each of the spines 214. Each tadpole cut-out 211 is defined by an aperture cut-out 212B that is merged with grooved cut-out 212C. It is noted that while aperture 212A or 212B is shown approximating the of a circle, it is possible to have cut-out opening 212A or 212B in any shape as long as each aperture 212A or 212B has the requisite negative area. In the event the aperture 212A is configured as a circle, aperture 212A has central void A0(of negative area) that can be approximated by a first virtual circle with radius r0 while each aperture 212B has a second area A2 that can be represented by a second virtual circle with radius r2. The apertures 212B (or the "heads" of the tadpole cut-outs) are radially arrayed so that apertures 212B are contiguous to a first virtual circle with radius r1. The second virtual circle may have a second radius r2 of 1.2 times that of the radius r0 of the first virtual circle representing aperture 212A while the first virtual circle r1 may have radius r1 of approximately 1.5 times that of the radius of the central virtual circle r0. The tail or grooved opening 212C of the "tails" extends towards the proximal end of the basket 38 for a length L1 so that each tail is contiguous to an inside circumference of a third virtual circle 215. Slot length L1 includes approximately 6-10 times that of the first radius r1. Third virtual circle 215 may have a radius r3 extending from the longitudinal axis 86 where radius r3 includes approximately 10-15 times that of either first radius r1 or central radius r0. In the exemplary embodiment (amongst many), the negative area of each of the tadpole cut-out 211 includes approximately 0.2 mm-squared while the negative area of center aperture 212A includes approximately 0.05mm-squared so that the total negative area defined by all of the cut-outs includes approximately 1.4mm-squared. In the same exemplary embodiment, the central radius r0 may be approximately 0.13mm, the second radius r2 may be approximately 0.2mm, and the first radius r1 may be approximately 0.23mm.

In FIG. 6D, the design of the basket 38 is provided with an aperture 212A at approximate center (i.e., axis 86) of the spines 214. Each spine 214 is provided a comet-shaped cut-out 211 with head portion 212B with an open tapered slot tail 212C tapering towards the proximal portion of each spine 214. The comet-shaped cut-outs 212B are arrayed so that the distal head portion 212B of the cut-out 211 are contiguous to an outside circumference of second virtual circle 213 while the proximal slotted opening 212C of the cut-outs 211 are contiguous on the inside circumference of third virtual circle 215. Where the aperture 212A is configured as a circular hole located on central axis 86 with radius r0 where the first radius r1 includes approximately 90% of the central radius r0, the second virtual circle 213 may have a second radius r2 of approximately 2.5 times that of central radius r0 while the third virtual circle 215 has a radius r3 of approximately 10 times that of the central radius r0 (all measured from center axis 86). Spine 214 has a first width W1 that tapers towards central axis 86 to a narrower second spine width W2 of approximately 66% of first spine width W1 at its narrowest point before being sub-divided by comet shaped cut-out 212B into two narrower spine arms with each arm including a third spine width W3 of approximately 1/3 that of the width W1. The comet shaped cut-out 212B has a length L1 along the spine of approximately 1.8 times that of the largest spine width W1.

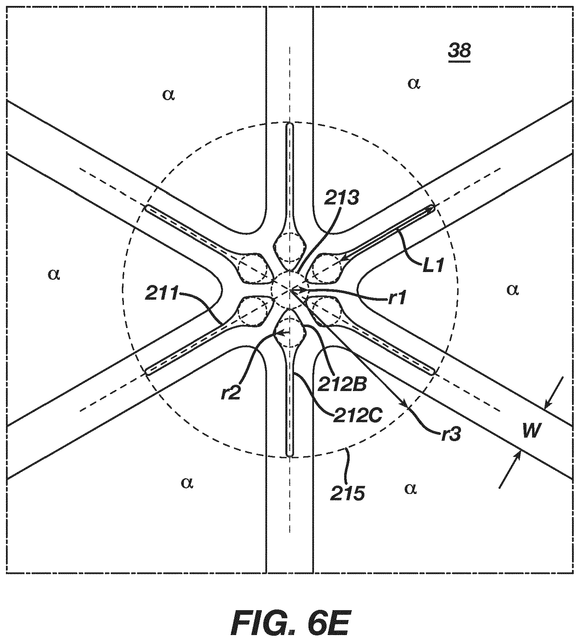

In FIG. 6E, the center (on axis 86) of the radiating spines 214 for basket 38 does not have a cut-out so that there is no void at the center of the basket to act as sharp edge surface (at the edge of such center aperture) against biological tissues. To allow for consistent folding of the spines near the distal portion of basket 38, each spine is provided with a tadpole shaped cut-out 211 that extends from the head portion 212B to tail portion 212C. The head portions 212B are arrayed so that the head portions 212B are contiguous to an outside circumference of first virtual circle 213 with radius r1. Each head portion 212B has a negative surface area that can be approximated by a second virtual circle with radius r2 of approximately 90% of the first radius r1. The tail portions 212C are bounded by a third virtual circle 215 with a radius r3 approximately 10 times that of the first radius. The length L1 of each of the tail portion includes approximately 1.5 times that of the width W1 of the spine 214. In one exemplary embodiment (out of many), the total negative area of the six cut-outs includes approximately 1.5 mm-squared.

The spines 214 can be folded or otherwise bent such that each respective attachment end 216 of the spine 214 can be inserted into the distal end 85 of the tubular shaft 84 (as shown in FIG. 2B) and relief lands 96 of spine retention hub 90 (not shown). Although not shown in FIGs. 5A and 5B, it will be appreciated that electrodes 40 can be attached to spines 214 before the spines are inserted into the tubular shaft 84 to form the basket assembly 38. As stated previously, the spines 214 can include a flexible, resilient material (e.g., a shape-memory alloy such as nickel-titanium, also known as Nitinol) that can enable the basket assembly 38 to transition to its expanded form (as shown in FIG. 2A) when the basket assembly 38 is deployed from tubular shaft 84. As will become apparent throughout this disclosure, spines 214 can be electrically isolated from electrode 40 to prevent arcing from electrode 40 to the respective spine 214.

As will be appreciated by one skilled in the art with the benefit of this disclosure, basket assembly 38 shown in FIGs. 2A through 2C including spines 214 formed from a single sheet of planar material and converging at a central intersection is offered merely for illustrative purposes and the disclosed technology can be applicable to other configurations of basket assemblies 38. For example, the described configuration of the basket spine assemblies can be obtained via laser cutting a nitinol tube and heat treating the spines from the tubular stock into substantially the planar form shown herein. As well, the disclosed technology can be applicable to basket assemblies 38 formed from a single spine 214 or multiple spines 214 with each spine 214 being attached at both ends. In other examples, the basket assembly 38 can include a central hub connecting the multiple spines 214 together at a distal end 39 of the basket assembly 38. In yet other examples, the basket assembly 38 can include a single spine 214 configured to form a spiral, multiple spines 214 configured to form a spiral, multiple spines 214 configured to form a tripod or multiple tripods, or any other shape of basket assembly 38. Thus, although FIGs. 2A through 2C illustrate a specific configuration of basket assembly 38, the disclosed technology should not be construed as so limited.

In the exemplary embodiments shown herein, the spines width W may have a nominal width of approximately 0.6 mm and can be as low as 0.2 mm or as large as 1.5 mm. The thickness of each spine can be nominally 0.09 mm and can vary from 0.05mm to 0.2mm. It should be noted that these values for width and thickness can vary depending on the stiffness desired.

Referring back to FIG. 2A through FIG. 2C, one or more electrodes 40 can be attached to spines 214 to form the basket assembly 38. In some examples, each electrode 40 can include electrically conductive material (e.g., gold, platinum and palladium (and their respective alloys)).

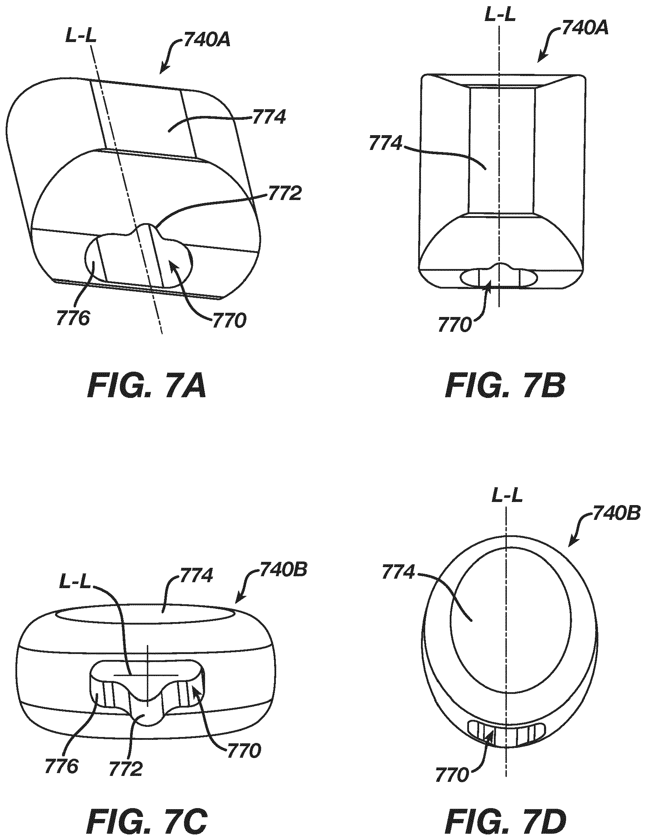

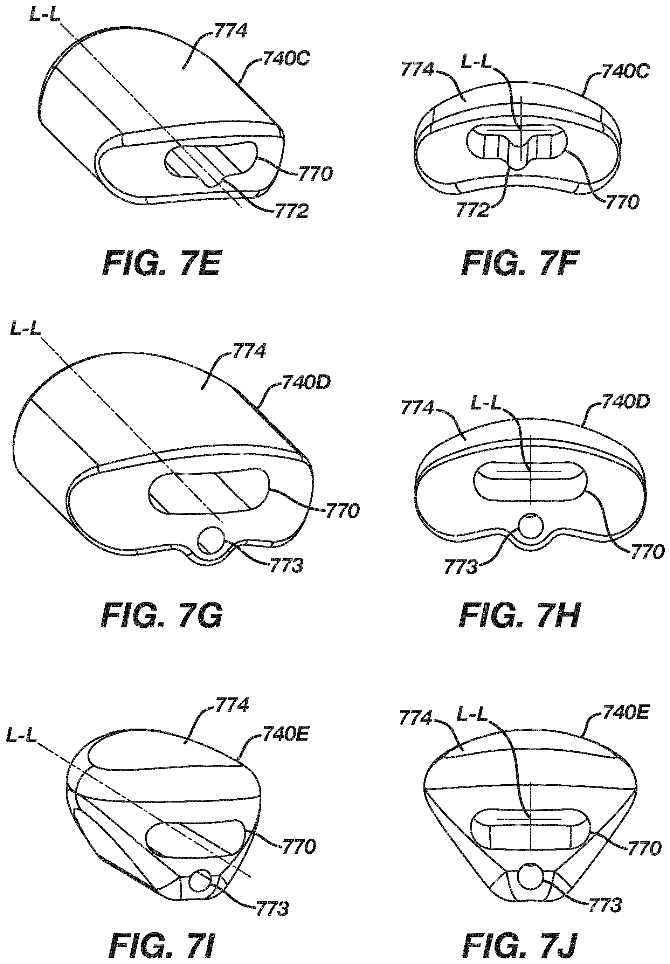

Turning to FIGs. 7A through 7J, electrode 40 can have a variety of cross-sectional shapes, curvatures, lengths, lumen number and lumen shape as provided as examples in electrodes 740A-740E. The electrodes 740A-740E are offered to illustrate various configurations of electrodes 40 that can be used with the medical device 22 but should not be construed as limiting. One skilled in the art will appreciate that various other configurations of electrodes 40 can be used with the disclosed technology without departing from the scope of this disclosure.

Each electrode 740A-740E can have an outer surface 774 facing outwardly from electrode 740 and an inner surface 776 facing inwardly toward electrode 740 where at least one lumen 770 is formed through electrode 740. The lumen 770 can be sized and configured to receive a spine 214 such that spine 214 can pass through electrode 740. Lumen 770 can be a symmetric opening through electrode 740A-740E and can be disposed offset with respect to a longitudinal axis L-L of the respective electrode. In other examples, lumen 770 can pass through electrode 740 in a generally transverse direction with respect to the longitudinal axis L-L of the respective electrode. Furthermore, lumen 770 can be positioned in electrode 740 nearer a bottom surface, nearer a top surface, or nearer a middle of electrode 740 depending on the particular configuration. In FIGs. 7A, 7C, and 7E through 7J, the top surface (upper side) is oriented toward the top of the drawing, the bottom surface (lower side) is oriented toward the bottom of the drawing, and the middle is between the top surface and the bottom surface. In other words, each electrode 740A-740E can include a lumen 770 that is offset with respect to a centroid of the electrode740A-740E.

In addition, as shown in FIGs. 7A through 7F, electrodes 740A-740C can have a wire relief 772 forming a recess or depression in electrode 740 adjacent lumen 770 for one or more wires to pass through lumen 770 along with a respective spine 214. Relief 772 can be sized to provide room for a wire of electrode 740 to pass through electrode 740 such that electrode 740 can be in electrical communication with the control console 24.

Alternatively, or in addition thereto, wires can pass through a wire lumen 773 as shown in example electrodes 740D and 740E in FIGs. 7G through 7J. Although not depicted, electrodes 40 may include both a wire relief 772 adjacent lumen 770 and wire lumen 773. Such electrode may permit additional wires to pass through the electrode body.

As shown in FIGs. 7A-7J, the electrodes 740A-740E can include various shapes depending on the application. For example, as illustrated in FIGs. 7A and 7B, the electrode 740A can have a substantially rectangular cuboid shape with rounded edges. In other examples, the electrode 740B can have a substantially ovoid shape (as illustrated in FIGs. 7C and 7D), the electrode 740C, 740D can have a contoured shape including a convex side and a concave side (as illustrated in FIGs. 7E through 7H), or the electrode 740E can have a contoured shape including substantially more material proximate an upper side than a lower side of the electrode 740E (as illustrated in FIGs. 7I and 7J). As will be appreciated by one of skill in the art, the various example electrodes 740A-740E shown in FIGs. 7A-7J, and described herein, are offered for illustrative purposes and should not be construed as limiting.

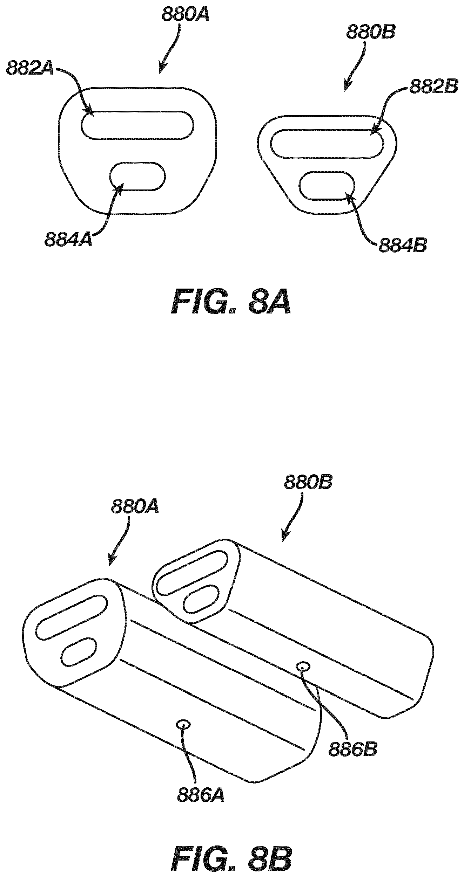

FIGs. 8A and 8B are schematic pictorial illustrations showing various insulative jackets 880A, 880B of a given medical device 22. FIG. 8A is a front view while FIG. 8B is a perspective view of insulative jackets 880A, 880B. Insulative jackets 880A, 880B can be made from a biocompatible, electrically insulative material such as polyamide-polyether (Pebax) copolymers, polyethylene terephthalate (PET), urethanes, polyimide, parylene, silicone. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets 880A, 880B may also include one or more additives or fillers, such as, for example, polytetrafluoroethylene (PTFE), boron nitride, silicon nitride, silicon carbide, aluminum oxide, aluminum nitride, zinc oxide, and the like. Insulative jacket 880A, 880B can help to insulate a spine 214 and/or wires passing through insulative jacket 880A, 880B from electrode 40 to prevent arcing from electrode 40 to the spine 214 and/or mechanical abrasion of wires passing through insulative jacket 880A, 880B.

As illustrated in FIGs. 8A and 8B, insulative jackets 880A, 880B, can include a cross-sectional shape that is substantially trapezoidal. The insulative jacket may consist of a single lumen or multi-lumen configuration. Multi-lumen jackets may be configured such that the alloy frame and wires share a single lumen while the second lumen may be used for irrigation. The alloy frame and wires may occupy separate lumens, also, as described. The current embodiment does not utilize irrigated jackets. For these designs, the insulative jackets may be continuous (individual sleeves extending from proximal to distal end of each alloy frame strut), segmented (bridging between electrode gaps), or a combination of both. Furthermore, insulative jacket 880A, 880B can include a first lumen 882A, 882B and a second lumen 884A, 884B. First lumen 882A, 882B can be configured to receive spine 214 while second lumen 884A, 884B can be configured to receive a wire, or vice-versa. In other examples, first lumen 882A, 882B and second lumen 884A, 884B can each be configured to receive one or more wires that can be connected to one or more electrodes 40. Furthermore, as illustrated in FIG. 8B, insulative jacket 880A, 880B can include an aperture 886A, 886B through which a wire can be electrically connected to electrode 40. Although illustrated in FIG. 8B as being proximate a bottom of insulative jacket 880A, 880B, aperture 886A, 886B can be positioned proximate a top or side of insulative jacket 880A, 880B. Furthermore, insulative jacket 880A, 880B can include multiple apertures 886A, 886B with each aperture being disposed on the same side of insulative jacket (i.e., top, bottom, left, right) or on different sides of the insulative jacket depending on the application.

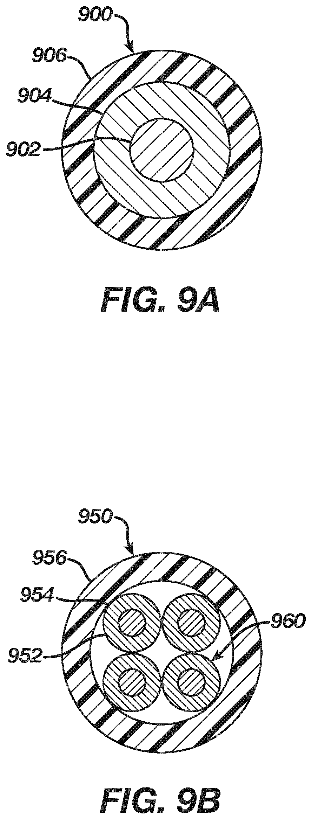

FIGs. 9A and 9B are schematic pictorial illustrations showing cross-sectional views of a given wire 900, 950 that can be connected to a given electrode 40, in accordance with an embodiment of the present invention. FIG. 9A illustrates a solid core wire 900. FIG. 9B illustrates a stranded wire 950. Each wire 900, 950 can extend through at least a portion of tubular shaft 84 and tubular shaft 84. Solid core wire 900 can include an electrically conductive core material 902 and an electrically conductive cover material 904 circumscribing electrically conductive core material 902. Likewise, stranded wire 950 can include strands each including an electrically conductive core material 952 and an electrically conductive cover material 954 circumscribing the electrically conductive core material 952. Each wire 900, 950 can include an insulative jacket 906 circumscribing the conductors. The wires 900, 950 can be configured to withstand a voltage difference of adjacent wires sufficient to deliver IRE pulses. Preferably, the wires 900, 950 can withstand at least 900V, and more preferably at least 1,800V between adjacent wires. To reduce likelihood of dielectric breakdown between conductors of adjacent wires, electrically conductive cover material 904, 954 can have a lower electrical conductivity compared to core material 902, 952.

Insulative jacket 906 can be configured to have a temperature rating between 150 and 200 degrees Centigrade so that the electrically insulative jacket 906 melts or degrades (e.g., chars and crumbles) during soldering of wire 900 to electrodes 40 (e.g., at a temperature of 300 degrees Centigrade) and therefore insulative jacket 906 of wire 900 does not need to be mechanically stripped. In other examples, insulative jacket 906 can have a temperature rating greater than 200 degrees Centigrade to prevent electrically insulating material 902 melting or degrading (e.g., charring and crumbling) during manufacture of medical probe 22 and/or during use. Insulative jacket 906 can be mechanically stripped from wire 900 prior to wires 900 being electrically connected to electrodes 40.

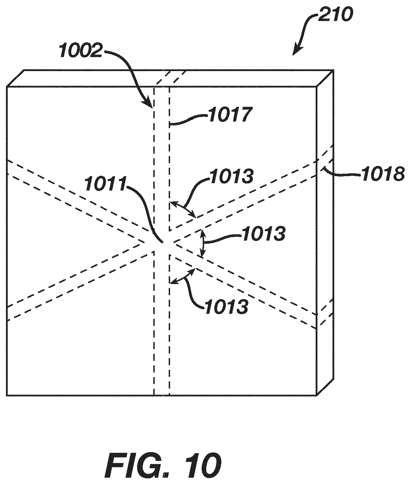

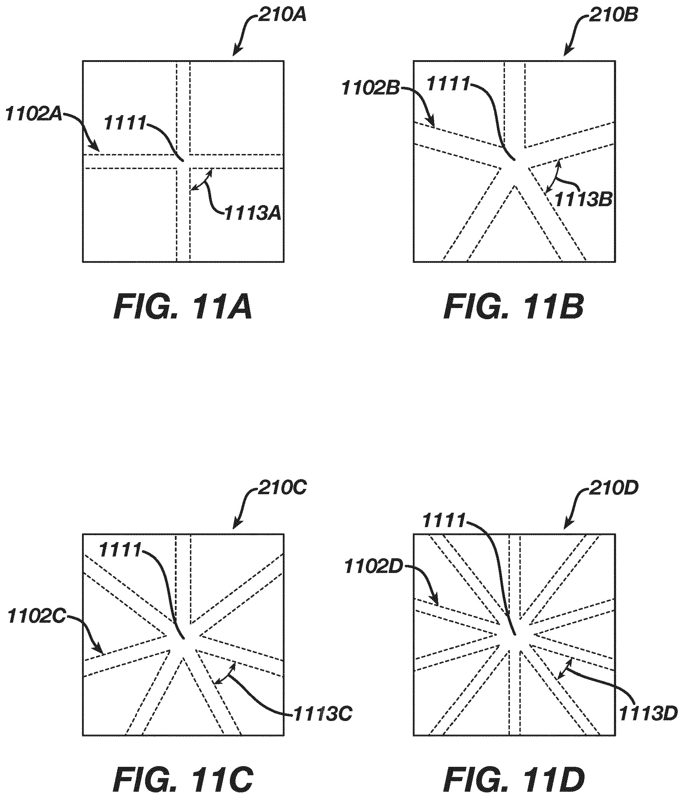

FIGs. 10-11D are schematic pictorial illustrations of cutting patterns for various linear spines patterns 1002 from a planar sheet of material 210. As described supra, planar sheet of material 210 can include a number of spines 214 ranging from about four to about ten spines. As illustrated in FIG. 10, planar sheet of material 210 can include central intersection 1011 and spine pattern 1002, which includes one or both of longitudinal scores 1017 and transverse scores 1018. In any of the embodiments disclosed herein, planar sheet of material 210 can also include a central intersection 1011 and spine patterns 1002 including an equiangular pattern 1013. Planar sheet of material 210 can include spine patterns including a number of spine patterns 1002 forming spines 214 in basket assembly 38. As would be understood by one of skill in the art, adjusting the number of spine patterns 1002 may impact a number of factors including, without limitation, stability, flexibility, surface contact, and ablation capacity of medical probe 22. FIGs. 11A through 11D provide example spine patterns 1102A, 1102B, 1102C, 1102D, although additional spine patterns are contemplated. Similar to the above planar sheet of material 210, spine patterns 1102A-1102D can include a respective central intersection 1111 and a respective equiangular pattern 1113A-1113D. As would be appreciated by one of skill in the art, as the number of spines added to spine pattern 1102A-1102D, the angle for equiangular pattern 1113A-1113D may change. In each of these examples provided, planar sheet of material 210A, 210B, 210C, 210D may also include central intersections and spine patterns including equiangular patterns. Although not depicted in FIG. 11A-11D, planar sheet of material 210A-210D can include one or both of longitudinal scores 1117 and transverse scores 1118.

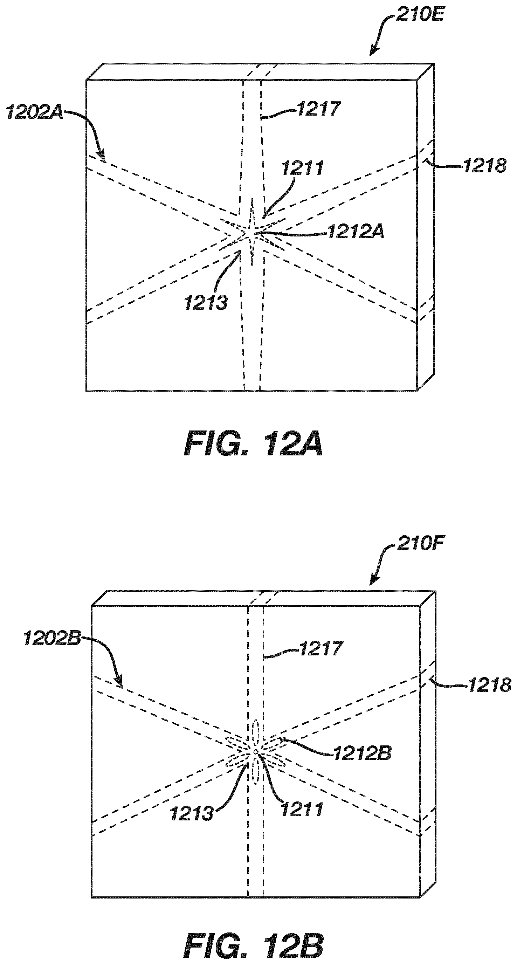

FIGs. 12A and 12B are schematic pictorial illustrations of cutting patterns for various linear spine patterns including one or more cutouts at a central spine intersection from a planar sheet of material. As described supra, planar sheet of material 210E, 210F may include a spine pattern 1202A including one cutout 1212A at central intersection 1211 or a spine pattern 1202B including two or more cutouts 1212B at central intersection 1211. As illustrated in FIGs. 12A and 12B, planar sheet of material 210E and 210F can include one or both of longitudinal scores 1217 and transverse scores 1218.

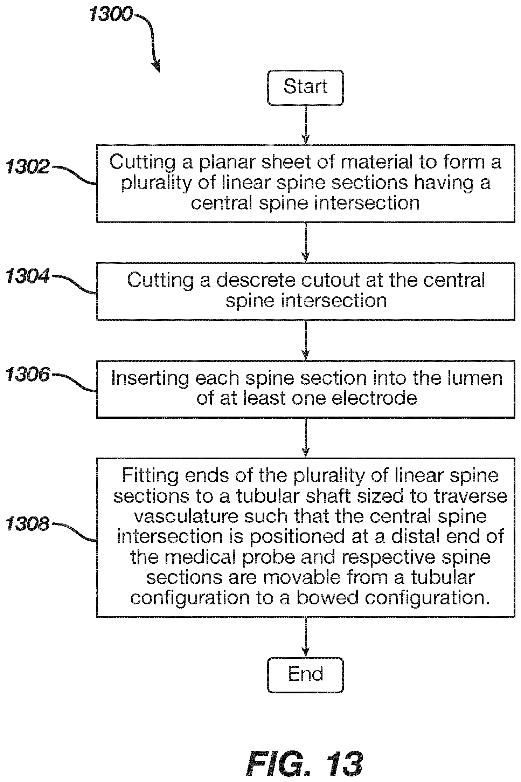

FIG. 13 is a flowchart illustrating a method 1300 of manufacturing a basket assembly 38, in accordance with an embodiment of the present invention. Method 1300 can include cutting 1302 a planar sheet of material 210 to form a plurality of linear spines 214 including a central spine intersection 211. Cutting 1302 the plurality of linear spines 214 can include cutting from a pattern 1002 (or 1102A-1102D) including longitudinal and transverse scores 1017, 1018. The planar sheet of resilient material can include shape-memory alloy such as nickel-titanium (also known as Nitinol), cobalt chromium, or any other suitable material. Method 1300 can include cutting 1304 a discrete cutout 214 at the central spine intersection 211. As described supra, the discrete cutout 214 can be a single cutout or two or more cutouts. In addition, the one or more discrete cutouts can be cut in a pattern to extend along at least a portion of each spine. In some examples, steps 1302 and 1304 may occur as simultaneous steps or as a sequence of steps. As an alternative to steps 1302 and 1304, metallic strands can be shaped similar to the pattern formed by cutting the planar sheet in steps 1302 and 1304.

Method 1300 can include inserting 1306 each spine into a lumen of at least one electrode. The electrodes can be positioned such that the electrodes are offset between electrodes on adjacent spines. Method 1300 can include fitting 1308 ends of the plurality of linear spines to a tubular shaft sized to traverse vasculature such that the central spine intersection is positioned at a distal end of the medical probe and respective spines are movable from a tubular configuration to a bowed configuration. As will be appreciated by one of skill in the art including the benefit of this disclosure, fitting 1308 an end of the spine into a tubular shaft can include attaching the spine 214 to a spine retention hub 90. Furthermore, the spine retention hub 90 and/or the spine 214 and the tubular shaft 84 can be inserted into a flexible insertion tube 30 to form the medical probe 22

In some examples, the method can also include forming an approximately spheroid or oblate-spheroid shape with the linear spines. Method 1300 can further include electrically connecting the wire to the one or more electrodes. Method 1300 can also include disposing an insulative sleeve over the linear spines and within the lumen of the respective electrode.

As will be appreciated by one skilled in the art, method 1300 can include any of the various features of the disclosed technology described herein and can be varied depending on the particular configuration. Thus, method 1300 should not be construed as limited to the particular steps and order of steps explicitly described herein. It is noted that while the preference for the exemplary embodiments of the medical probe is for IRE or PFA, it is possible to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

The embodiments described above are cited by way of example, and the present invention is not limited by what has been particularly shown and described hereinabove. Rather, the scope of the invention is defined by the claims and includes both combinations and sub combinations of the various features described and illustrated hereinabove, as well as variations and modifications thereof which fall within the scope of the claims.

## Claims

1. A medical probe (22), comprising:
a tubular shaft (84) including a proximal end and a distal end, the tubular shaft extending along a longitudinal axis;
an expandable basket assembly (38) proximate the distal end of the tubular shaft, the basket assembly comprising a single unitary structure that includes a plurality of linear spines (214) formed from a planar sheet of material (210), the spines converging at a central spine intersection (211), the central spine intersection including one or more cutouts (212) that allows for bending of the spines, each spine including a respective end connected to the distal end of the tubular shaft, the central spine intersection being positioned on the longitudinal axis at a distal end of the basket assembly; and
one or more electrodes (40) coupled to each of the spines, each electrode defining a lumen through the electrode so that a spine extends through the lumen of each of the one or more electrodes.

2. The medical probe according to claim 1, wherein the plurality of linear spines extend from the central spine intersection in an equiangular pattern such that respective angles between respectively adjacent spines are approximately equal.

3. The medical probe according to claim 1 or claim 2, further comprising a spine retention hub (90) disposed proximate the distal end of the tubular shaft, the spine retention hub comprising:
a cylindrical member (94) including a plurality of relief lands (96) disposed on an outer surface of the cylindrical member to allow each spine to be fitted into a relief land and retained therein; and
at least one electrode (99) disposed at a distal portion of the retention hub.

4. The medical probe according to any preceding claim, wherein the expandable basket assembly comprises at least one discrete cutout (214) located proximate the central spine intersection.

5. The medical probe according to any preceding claim, wherein the one or more cutouts extend along at least a portion of each spine.

6. The medical probe according to any preceding claim, wherein the one or more electrodes are configured to deliver electrical pulses for irreversible electroporation, the pulses including a peak voltage of at least 900 volts, V.

7. A method (1300) of constructing a medical probe according to any of the preceding claims, the method comprising:
cutting (1302) a planar sheet of material (210) to form a plurality of linear spines (214) including a central spine intersection (211);
cutting (1304) a discrete cutout (214) at the central spine intersection;
inserting (1306) each spine into a lumen of at least one electrode; and
fitting (1308) ends of the plurality of linear spines to a tubular shaft sized to traverse vasculature such that the central spine intersection is positioned at a distal end of the medical probe and respective spines are movable from a tubular configuration to a bowed configuration.

8. The method according to claim 7, wherein cutting the plurality of linear spines comprises cutting from a pattern (1002) comprising longitudinal (1017) and transverse (1018) scores.

9. The method according to claim 7 or claim 8, further comprising cutting at least two cutouts at the central spine intersection.

10. The method according to any of claims 7 to 9, further comprising offsetting the electrodes between adjacent spines.

11. The method according to any of claims 7 to 10, wherein each electrode comprises a relief adjacent the lumen to allow a wire to extend adjacent to the lumen.

12. The method according to claim 11, further comprising electrically connecting the wire to the electrodes.

13. The method according to any of claims 7 to 12, further comprising disposing an insulative sleeve over the linear spines and within the lumen of the respective electrode.

## Patentansprüche

1. Medizinische Sonde (22), umfassend:
einen rohrförmigen Schaft (84), einschließlich eines proximalen Endes und eines distalen Endes, wobei sich der rohrförmige Schaft entlang einer Längsachse erstreckt;
eine expandierbare Korbanordnung (38) nahe dem distalen Ende des röhrenförmigen Schafts, wobei die Korbanordnung eine einzige einheitliche Struktur umfasst, die eine Vielzahl von linearen Streben (214) einschließt, die aus einem planaren Materialblatt (210) gebildet sind, wobei die Streben an einer zentralen Strebenschnittstelle (211) zusammenlaufen, wobei die zentrale Strebenschnittstelle einen oder mehrere Ausschnitte (212) einschließt, die ein Biegen der Streben ermöglichen, wobei jede Strebe ein jeweiliges Ende einschließt, das mit dem distalen Ende des röhrenförmigen Schafts verbunden ist, wobei die zentrale Strebenschnittstelle auf der Längsachse an einem distalen Ende der Korbanordnung positioniert ist; und
eine oder mehrere Elektroden (40), die mit jeder der Streben gekoppelt sind, wobei jede Elektrode ein Lumen durch die Elektrode definiert, so dass sich eine Strebe durch das Lumen jeder der einen oder der mehreren Elektroden erstreckt.

2. Medizinische Sonde nach Anspruch 1, wobei sich die drei linearen Streben von der zentralen Strebenschnittstelle in einem gleichwinkligen Muster derart erstrecken, dass die jeweiligen Winkel zwischen jeweils benachbarten Streben etwa gleich sind.

3. Medizinische Sonde nach Anspruch 1 oder Anspruch 2, ferner umfassend eine Strebenrückhaltenabe (90), die proximal zum distalen Ende des röhrenförmigen Schafts angeordnet ist, wobei die Strebenrückhaltenabe umfasst:
ein zylindrisches Element (94), einschließlich einer Vielzahl von Entlastungsflächen (96), die auf einer Außenoberfläche des zylindrischen Elements angeordnet sind, um zu ermöglichen, dass jede Strebe in eine Entlastungsfläche eingepasst und darin gehalten wird; und
mindestens eine Elektrode (99), die an einem distalen Abschnitt der Rückhaltenabe angeordnet ist.

4. Medizinische Sonde nach einem der vorstehenden Ansprüche, wobei die expandierbare Korbanordnung mindestens einen diskreten Ausschnitt (214) umfasst, der sich in der Nähe der zentralen Strebenschnittstelle befindet.

5. Medizinische Sonde nach einem der vorstehenden Ansprüche, wobei sich die eine oder die mehreren Aussparungen entlang mindestens eines Abschnitts jeder Strebe erstrecken.

6. Medizinische Sonde nach einem der vorstehenden Ansprüche, wobei die eine oder die mehreren Elektroden konfiguriert sind, um elektrische Impulse für eine irreversible Elektroporation abzugeben, wobei die Impulse eine Spitzenspannung von mindestens 900 Volt, V, einschließen.

7. Verfahren (1300) zum Herstellen einer medizinischen Sonde nach einem der vorstehenden Ansprüche, wobei das Verfahren umfasst:
Schneiden (1302) eines planaren Materialblatts (210) zur Bildung einer Vielzahl von linearen Streben (214) einschließlich einer zentralen Strebenschnittstelle (211);
Schneiden (1304) eines diskreten Ausschnitts (214) an der zentralen Strebenschnittstelle;
Einführen (1306) jeder Strebe in ein Lumen mindestens einer Elektrode; und
Montieren (1308) von Enden der Streben der Vielzahl von Streben an einen rohrförmigen Schaft, der bemessen ist, um ein Gefäßsystem derart zu durchqueren, dass die zentrale Strebenschnittstelle an einem distalen Ende der medizinischen Sonde positioniert ist und die jeweiligen Streben von einer rohrförmigen Konfiguration in eine gebogene Konfiguration bewegbar sind.

8. Verfahren nach Anspruch 7, wobei das Schneiden der Vielzahl von linearen Streben das Schneiden nach einem Muster (1002) umfasst, das Längs-(1017) und Quer- (1018) Einschnitte umfasst.

9. Verfahren nach Anspruch 7 oder 8, ferner umfassend das Schneiden von mindestens zwei diskreten Ausschnitten an der zentralen Strebenschnittstelle.

10. Verfahren nach einem der Ansprüche 7 bis 9, ferner umfassend das Versetzen der Elektroden zwischen benachbarten Streben.

11. Verfahren nach einem der Ansprüche 7 bis 10, wobei jede Elektrode eine Entlastung benachbart zu dem Lumen umfasst, um zu ermöglichen, dass sich ein Draht benachbart zu dem Lumen erstreckt.

12. Verfahren nach Anspruch 11, ferner umfassend das elektrische Verbinden des Drahts mit den Elektroden.

13. Verfahren nach einem der Ansprüche 7 bis 12, ferner umfassend das Anordnen einer isolierenden Hülse über den linearen Streben und innerhalb des Lumens der jeweiligen Elektrode.

## Revendications

1. Sonde médicale (22), comprenant :
une tige tubulaire (84) comportant une extrémité proximale et une extrémité distale, la tige tubulaire s'étendant le long d'un axe longitudinal ;
un ensemble panier expansible (38) à proximité de l'extrémité distale de la tige tubulaire, l'ensemble panier comprenant une structure d'un seul tenant qui comporte une pluralité d'éléments d'armature linéaires (214) formés à partir d'une feuille plane de matériau (210), les éléments d'armature convergeant au niveau d'une intersection centrale d'éléments d'armature (211), l'intersection centrale d'éléments d'armature comportant une ou plusieurs découpes (212) qui permettent le cintrage des éléments d'armature, chaque élément d'armature comportant une extrémité respective reliée à l'extrémité distale de la tige tubulaire, l'intersection centrale d'éléments d'armature étant positionnée sur l'axe longitudinal au niveau d'une extrémité distale de l'ensemble panier ; et
une ou plusieurs électrodes (40) couplées à chacun des éléments d'armature, chaque électrode définissant une lumière à travers l'électrode de sorte qu'un élément d'armature s'étend à travers la lumière de la ou de chaque électrode.

2. Sonde médicale selon la revendication 1, dans laquelle la pluralité d'éléments d'armature linéaires s'étendent à partir de l'intersection centrale d'éléments d'armature dans un motif équiangulaire de telle sorte que des angles respectifs entre des éléments d'armature respectivement adjacents sont approximativement égaux.

3. Sonde médicale selon la revendication 1 ou la revendication 2, comprenant en outre une noix de rétention d'éléments d'armature (90) disposée à proximité de l'extrémité distale de la tige tubulaire, la noix de rétention d'éléments d'armature comprenant :
un élément cylindrique (94) comportant une pluralité de zones de soulagement (96) disposées sur une surface externe de l'élément cylindrique pour permettre à chaque élément d'armature de s'intégrer dans une zone de soulagement et d'y être retenu ; et
au moins une électrode (99) disposée au niveau d'une partie distale de la noix de rétention.

4. Sonde médicale selon l'une quelconque revendication précédente, dans laquelle l'ensemble panier expansible comprend au moins une découpe discrète (214) localisée à proximité de l'intersection centrale d'éléments d'armature.

5. Sonde médicale selon l'une quelconque revendication précédente, dans laquelle la ou les découpes s'étendent le long d'au moins une partie de chaque élément d'armature.

6. Sonde médicale selon l'une quelconque revendication précédente, dans laquelle la ou les électrodes sont configurées pour délivrer des impulsions électriques pour une électroporation irréversible, les impulsions comportant une tension de crête d'au moins 900 volts, V.

7. Procédé (1300) de construction d'une sonde médicale selon l'une quelconque des revendications précédentes, le procédé comprenant :
la découpe (1302) d'une feuille plane de matériau (210) pour former une pluralité d'éléments d'armature linéaires (214) comportant une intersection centrale d'éléments d'armature (211) ;
la découpe (1304) d'une découpe discrète (214) au niveau de l'intersection centrale d'éléments d'armature ;
l'insertion (1306) de chaque élément d'armature dans une lumière d'au moins une électrode ; et
l'ajustement (1308) d'extrémités de la pluralité d'éléments d'armature linéaires à une tige tubulaire dimensionnée pour traverser une vascularisation de telle sorte que l'intersection centrale d'éléments d'armature est positionnée au niveau d'une extrémité distale de la sonde médicale et que des éléments d'armature respectifs peuvent être déplacés d'une configuration tubulaire à une configuration arquée.

8. Procédé selon la revendication 7, dans lequel la découpe de la pluralité d'éléments d'armature linéaires comprend la découpe à partir d'un motif (1002) comprenant des entailles longitudinales (1017) et transversales (1018).

9. Procédé selon la revendication 7 ou la revendication 8, comprenant en outre la découpe d'au moins deux découpes au niveau de l'intersection centrale d'éléments d'armature.

10. Procédé selon l'une quelconque des revendications 7 à 9, comprenant en outre le décalage des électrodes entre des éléments d'armature adjacents.

11. Procédé selon l'une quelconque des revendications 7 à 10, dans lequel chaque électrode comprend un relief adjacent à la lumière pour permettre à un fil de s'étendre adjacent à la lumière.

12. Procédé selon la revendication 11, comprenant en outre la connexion électrique du fil aux électrodes.

13. Procédé selon l'une quelconque des revendications 7 à 12, comprenant en outre la mise en place d'un manchon isolant par-dessus les éléments d'armature linéaires et au sein de la lumière des électrodes respectives.
